# EUROPEAN PATENT APPLICATION

(11) **EP 1 820 498 A2**
(43) Date of publication of application: **22.08.2007**
(21) Application number: 07250689.2
(22) Date of filing: 20.02.2007
(51) Int. Cl.: A61K 9/52

(54) **Enteric-coated preparation for site-specific delivery of drug to site within the small intestine**

(30) Priority: 21.02.2006 JP 2006043752; 21.07.2006 JP 2006199255
(71) Applicant: Shin-Etsu Chemical Co., Ltd., Tokyo (JP)
(72) Inventor: Kusaki, Fumie, c/oSpeciality Chemicals Res. Cnter, Kubiki-ku Joetsu-shi, Niigata-ken (JP); Kokubo, H., c/oSpeciality Chemicals Res. Cnter, Kubiki-ku Joetsu-shi, Niigata-ken (JP); Sakuma, Shinji, Hirakata-shi, Osaka (JP); Yamashita, Shinji, Hirakata-shi, Osaka (JP)
(74) Representative: Denmark, James Christopher

(57) **Abstract**

Provided is an enteric-coated preparation comprising an enteric coating material capable of delivering a drug to a site within the small intestine in a site-specific manner. More specifically, provided is an enteric-coated preparation for delivering a drug to a site within the small intestine in a site-specific manner, comprising a drug-containing preparation and an enteric coating material which covers the preparation, wherein, in Dissolution Test as specified in the Japanese Pharmacopoeia Fourteenth Edition, no dissolution of the drug is observed in the Japanese Pharmacopoeia first fluid (pH 1.2) while dissolution of the drug is observed in the Japanese Pharmacopoeia second liquid (pH 6.8) 20 minutes or later from start of the Dissolution Test. The enteric coating material is preferably selected from the group consisting of hydroxypropylmethyl cellulose acetate succinate, hydroxypropylmethyl cellulose phthalate, methacrylic acid copolymer and carboxymethylethyl cellulose.

## Description

### BACKGROUND OF THE INVENTION

### 1. Field of the Invention

The present invention relates to preparation of an enteric-coated preparation which, after orally administered, does not release a drug contained therein during the retention in the stomach and just after passing through the stomach but delivers it specifically to a certain site in the small intestine. The present invention also pertains to a preparation process of an enteric-coated preparation which has been covered with an enteric coating material designed to accomplish the above-described drug delivery.

### 2. Description of the Related Art

Enteric coating has been used widely for various purposes. For example, it has been used mainly for protecting acid-susceptible drugs from gastric acid or protecting the gastric mucosa from irritation or damage resulting from drugs. For the purpose of realizing such enteric coating, a preparation design has been proposed in consideration of the physical and physiological environment in the human digestive tracts and passing time in the gastrointestinal tracts. In the gastrointestinal tracts of healthy persons, the physiological pH changes usually from acidity (pH of from about 1.8 to 4.5) to neutral (pH of from about 6.5 to 8.0) from the stomach to the small intestine and no difference is presumed to exist in the physiological environment between the small intestine and large intestine. The retention time of the preparation in the human stomach is from 0.5 to 10 hours although it differs greatly, depending on persons. The retention time of the preparation in the human stomach is also influenced by the eating condition or size of the preparation to be administered. On the other hand, the fluctuations of a passing time through the small intestine are relatively small and the passing time is said to be usually 3 ± 1 hours.

Various studies have been made on a method of releasing or delivering a drug selectively to a specific site in the small intestine. In addition to classic methods of using enteric-coated preparations or sustained release preparations (Chemical & Pharmaceutical Bulletin, 40, 3035-3041(1992)), enteric-coated sustained-release preparations and time-limited release enteric-coated preparations have been proposed (Japanese Patent No. 3185206, Japanese Patent Application Unexamined Publication No. 07-089849/1995 and WO 01-23000). However, the conventional methods have defects such as insufficient site selectivity, limitation in a usable active ingredient owing to the stability in the presence of an acid added to a coating layer (Japanese Patent Application Unexamined Publication No. 07-089849/1995 and WO 01-23000), and complex structure of the preparation which disturbs its industrialization (Japanese Patent No. 3185206). For example, sustained release preparations have the drawback that since a drug contained therein is released continuously, significant release occurs during the retention of the preparation in the stomach and during the passage of the small intestine. Enteric-coated preparations also have the drawback that although the release of the drug in the stomach is suppressed, the release is started abruptly during the transition from the stomach to the upper small intestine, which causes loss of a large portion of the drug by absorption or decomposition before the drug reaches the target site in the small intestine.

Moreover, it has recently been found that gastrointestinal absorption of some drugs is adversely affected by a diet. Many drugs have so far showed high absorption after diet (positive influence of diet), but some pharmaceuticals marketed or developed recently show low absorption after diet (negative influence of diet). Patients feel some inconvenience in taking drugs, for example, when their diet is restricted or they have to take a drug between meals. Such a limitation in administration method imposes a constant burden to patients who have to take a drug for chronic disease, leading to deterioration in compliance.

Most of dietary components are absorbed in the stomach and the upper and middle small intestines so that no dietary component may remain in the lower small intestine and in the vicinity of the large intestine. Since many drugs are presumed to exhibit equal drug absorption at the lower small intestine and at the upper small intestine, selective delivery of the drug in the middle or lower small intestines therefore enables effective absorption of the drug in the body without influence of a diet.

### SUMMARY OF THE INVENTION

With the foregoing in view, the present invention has been completed. An object of the present invention is to provide an enteric-coated preparation by using an enteric coating material capable of delivering a drug specifically to a target site in the small intestine.

The present inventors have carried out an extensive investigation to overcome the above-described problem. As a result, it has been found that regulating dissolution properties, at a specific pH, of an enteric-coated preparation obtained by applying enteric coating to a solid preparation while utilizing the pH-dependent solubility of an enteric polymer and combining the enteric polymer and an optional excipient or the like, the preparation disintegrates smoothly at a specific site inside the small intestine so that delivery of the drug can be controlled, leading to the completion of the invention.

The enteric-coated preparation of the present invention can deliver the drug therein to a specific site (middle or lower small intestines) in the small intestine in the living body. Accordingly, the protection of the drug in the stomach, absorption of the drug in the body and bioavailability can be improved. Delivery of the drug to the lower small intestine where only a small amount of dietary components remains can reduce the negative influence of the diet and improve the compliance of patients.

### BRIEF DESCRIPTION OF THE DRAWINGS

FIG. 1 illustrates the dissolution profile in Japanese Pharmacopoeia second fluid;
FIG. 2 illustrates the dissolution profile in a phosphate buffer having a pH of 7.6;
FIG. 3 illustrates the profile of the blood level in Example 1; and
FIG. 4 illustrates the profile of the blood level in Example 3.

### DETAILED DESCRIPTION OF THE PREFERRED EMBODIMENTS

The present invention will hereinafter be described specifically.

The enteric-coated preparation of the present invention is an orally administrable enteric-coated preparation comprising an inner core comprising a drug therein and a film layer comprising an enteric polymer designed for the site-specific delivery of the drug in the small intestine. Provided is the enteric-coated preparation of the present invention which has been covered with an enteric coating material for the site-specific delivery of the drug in the small intestine, wherein, in Dissolution Test as specified in The Japanese Pharmacopoeia Fourteenth Edition, no dissolution of the drug is observed in the Japanese Pharmacopoeia first fluid (pH 1.2) while dissolution of the drug is observed in the Japanese Pharmacopoeia second fluid (pH 6.8) 20 minutes or later from start of the Dissolution Test.

It has been considered that in the enteric-coated preparations comprising an enteric polymer having pH-dependent solubility, the dissolution properties of a drug directly reflect the solubility of the enteric polymer material. However, as a result of the investigation of the present invention, it has been found that the pH solubility of the enteric polymer material and dissolution properties of the resulting preparation do not always coincide with the drug releasability of the enteric film preparation in the actual living body, though there is some correlation between them. This suggests that detailed specification of these conditions which have not been analyzed yet enables site-specific delivery of a drug in the actual living body.

The term "in Dissolution test (Method 1 and Method 2) as specified in the Japanese Pharmacopoeia, no dissolution of the drug is observed in Japanese Pharmacopoeia first fluid (pH 1.2)" means that when the test on six samples is conducted for 2 hours, a dissolution rate from each of these samples is 5% by weight or less. The number of revolutions of the paddle is 50 rpm. The term "dissolution of the drug is observed in the Japanese Pharmacopoeia second fluid (pH 6.8) 20 minutes or later from start of the Dissolution Test" means that the dissolution rate from each of the six samples ranges from 0 to 5% by weight for 20 minutes after the test is started. The dissolution starting time of the drug in the Japanese Pharmacopoeia second fluid is 20 minutes or later, preferably 60 minutes or later from the start of the test.

The enteric-coated preparation of the present invention delivers a drug to a specific site in the small intestine.
In order to realize the object of the present invention, the drug is released preferably downstream of the middle small intestine, that is, at any site from the middle small intestine to the jejunum or ileum. The drug is released most preferably at any site from the middle small intestine to the lower small intestine (from the jejunum to the ileum).

Although no particular limitation is imposed on the drug, the drug to be released at the above-described sites may be preferred. Examples may include therapeutic agents for virus diseases such as trientine hydrochloride, bisphosphonate bone metabolism improvers such as alendronate, α-glucosidase inhibitors, aldose reduction inhibitors, anti-virus HIV protease inhibitors, anti-virus HIV reverse transcriptase inhibitors, and drugs which usually need irregular administration so as to avoid influence of diet, for example, during the hunger before meal, between meals, at the time of rising and the like.

Examples of an object to be covered with the enteric coating material of the present invention may include the above-described drugs which have been granulated or tableted together with a binder such as hydroxypropylmethyl cellulose, methyl cellulose, hydroxypropyl cellulose or polyvinylpyrrolidone and/or an excipient such as corn starch, crystalline cellulose or lactose; particles obtained by layering the above-described drugs together with the binder and/or the excipient on spherical core particles such as sucrose, a mixture of sucrose and corn starch, crystalline cellulose, calcium phosphate or silica; and capsules having the above-described particles filled therein.

Examples of the enteric coating material may include celluloses such as cellulose acetate phthalate, cellulose acetate trimellitate, cellulose acetate succinate, methyl cellulose phthalate, hydroxymethylethyl cellulose phthalate, hydroxypropylmethyl cellulose phthalate, hydroxypropylmethyl acetate succinate, hydroxypropylmethyl acetate maleate, hydroxypropylmethyl trimellitate and carboxymethylethyl cellulose; vinyl such as polyvinyl butyrate phthalate and polyvinyl alcohol acetate phthalate; and acrylic such as copolymer of methacrylic acid and ethyl acrylate and copolymer of methacrylic acid and methyl methacrylate ("Eudragit L and S"; trade name).

According to the invention, the enteric coating material may preferably include hydroxypropylmethyl cellulose acetate succinate, hydroxypropylmethyl cellulose phthalate, carboxymethylethyl cellulose and copolymer of methacrylic acid and ethyl acrylate, and may further preferably include hydroxypropylmethyl cellulose acetate succinate and copolymer of methyl methacrylate and methacrylic acid. The hydroxypropylmethyl cellulose acetate succinate may be most preferable of them because compared with methyl methacrylate / methacrylic acid copolymer which usually employs a coating method of using an organic solvent, it can employ any coating method selected from various methods such as organic solvent coating, aqueous coating and dry coating, depending on the properties of an active ingredient.

The enteric coating material capable of delivering a drug to a target site can be prepared in a conventional preparation process. Many of the above-described enteric coating materials have a molecular structure containing an ionic dissociable group for exhibiting pH-dependent dissolution properties.

For example, when hydroxypropylmethyl cellulose acetate succinate is used as the enteric coating material, dissolution can be started at a predetermined pH by controlling the content of its dissociable group, that is, a succinoyl group. It is believed that pH inside the small intestine in the living body varies from about 4.5 to 8.0, depending on the site in the small intestine, more specifically, from the upper small intestine to the middle small intestine and to the lower small intestine. When a coating film comprising hydroxypropylmethyl cellulose acetate succinate is designed to be dissolved at the higher pH side, an enteric-coated preparation comprising the coating film is considered to release a drug at a site from the middle small intestine to the lower small intestine. In order to dissolve hydroxypropylmethyl cellulose acetate succinate at the high pH side, first of all, the succinoyl group content may be adjusted to preferably from 0.1 to 10% by weight, especially preferably from 3 to 8% by weight. In this case, as for the contents of the other substituents in the material, the content of a methoxyl group may be preferably from 12 to 28% by weight, more preferably from 20 to 26% by weight; the content of a hydroxypropoxyl group may be preferably from 4 to 23% by weight, more preferably from 5 to 10% by weight; and the content of an acetyl group may be preferably from 2 to 16% by weight, more preferably from 5 to 14% by weight.

When the enteric coating material is methyl methacrylate/methacrylic acid copolymer, methacrylic acid copolymer S described in "Japanese Pharmaceutical Excipients" may be preferably used. Its methacrylic acid content may be preferably from 25 to 34.5% by weight to satisfy the above-described solubility.

A ratio of the acetyl group content to the succinoyl group content may be preferably from 1.25 to 5, more preferably from 1.5 to 3.5. When the ratio is less than 1.25, dissolution may occur at the low pH side. When the ratio is more than 5, dissolution may not occur owing to an increase in hydrophobicity.

A desirable enteric coating material can be obtained by designing its molecular structure so that a single film formed by using the resulting enteric coating material disintegrates in a 0.1N aqueous NaOH solution (pH 13); does not disintegrate in the Japanese Pharmacopoeia second fluid within 1 hour, preferably within 1.5 hours; and disintegrates within 2 hours at a pH value of 7.0 or greater, more preferably within 2 hours at a pH value of 7.5 or greater.

For the preparation of the enteric-coated preparation of the present invention, the above-described enteric coating material may be used singly or with the admixture thereof.

Such an enteric coating material can be used for coating in form of a solution obtained by dissolving the polymer in an organic solvent, or in form of an aqueous latex or aqueous dispersion. It may also be used for dry coating in which polymer powder is fed directly to the object, while a plasticizer being simultaneously spayed.

The coating composition (coating solution) may preferably comprise, in addition to the enteric coating material, talc as an excipient. The talc may be added in an amount of from 1 to 100 parts by weight, preferably from 3 to 50 parts by weight based on 100 parts by weight of the enteric coating material.

The enteric coating composition can optionally comprise a plasticizer such as triethyl citrate, acetic and fatty acid esters of glycerol, triacetin or dibutyl phthalate; or a wax such as the following: liquid paraffin; microcrystalline wax; bees wax; a higher alcohol such as lauryl alcohol or cetyl alcohol; a higher fatty acid such as oleic acid, linoleic acid, palmitic acid or stearic acid; a higher fatty acid ester such as carnauba wax; olive oil; a glycerin fatty acid ester such as glycerin monostearate; or a sucrose fatty acid ester. The above-described plasticizer or wax may be comprised singly or with the admixture thereof. The plasticizer may preferably include triethyl citrate and acetic and fatty acid esters of glycerol. Furthermore, pharmaceutically acceptable drugs, surfactants (sodium lauryl sulfate, etc.) for improving the dispersibility of the composition, colorants, pigments or sweeteners may be added.

Conventional coaters may be used. For example, when spray coating is employed, a pan coater, a drum type coater, a fluidized bed coater or a stirred fluidized bed coater may be employed. As a spray device attached to these coaters, any one of an air spray, an airless spray, a three-fluid spray and the like may be used. When a dry spray is employed, any one of a centrifugal fluidizing coater, a pan coater, a fluidized bed coater, a centrifugal rotary fluidized bed coater and the like may be employed.

Enteric coating is applied to the inner core of tablets, granules or capsules comprising a drug while using the above-described enteric coating composition and coater in combination. After the coating, the coated product may be subjected to another treatment such as drying, heating, a high-gloss finish, application of a sugar coating or application of another coating material.

A preparation to be subjected to the coating of the present invention may include a tablet, a granule, a fine granule and a capsule. An amount of the coating composition to be applied to the surface of such a solid preparation may be variable depending on a type, a shape, a size or a surface condition of the preparation, or properties of a drug or an additive comprised by the preparation. The amount (weight) of the enteric coating material may be from 3 to 100% by weight, preferably from 10 to 100% by weight, especially preferably from 10 to 50% by weight based on the weight of the solid preparation. When the amount is less than 3% by weight, sufficient acid resistance and lag time may not be attained, which may not result in the site-specific release in the small intestine. When the amount is more than 100% by weight, the coat (film) may not completely dissolve in the intestines so that the preparation may reach the large intestine or be excreted without releasing the drug in the small intestine. Thus, the amounts outside the above-described range may not be preferable.

It is preferred that in Dissolution Test of the Japanese Pharmacopoeia Fourteenth Edition, the drug of the enteric-coated preparation thus prepared is eluded neither in the Japanese Pharmacopoeia first fluid nor purified water even after two-hour test and at the same time, the lag time of the preparation which is the time required for the drug to start to be released in the Japanese Pharmacopoeia second fluid is preferably 20 minutes or higher but less than 240 minutes, more preferably 30 minutes or higher but less than 120 minutes from the start of the test.

It has been found that the enteric-coated preparation having the above-described design can deliver the drug to a specific site in the small intestine in the living body of animals.

The present invention will hereinafter be described specifically by Examples and Comparative Examples. However, it should not be construed that the present invention is limited to or by them.

### Preparation of a sample piece for dissolution test

Enteric coating materials having pH-dependent solubility were prepared as shown in Table 1. A 100 µm thick film piece was prepared by glass casting of a 20 wt% solution (solvent: ethanol/water (weight ratio of 8/2)) of each of the enteric coating materials obtained in Examples 1 to 8 and Comparative Examples 1 to 3. The film piece thus obtained was cut into a 10 x 10 mm square and used as a sample for the dissolution test of a film.

The pH dependent solubility of each sample was evaluated based on the results of the disintegration test with n=6 in accordance with the "Disintegration Test" of the Japanese Pharmacopoeia Fourteenth Edition. Test fluids employed were the first fluid (pH 1.2) and the second fluid (pH 6.8). In addition, phosphate buffers of pH 7.6 and pH 8.0, or a borate buffer of pH 8.6, and further a 0.1N aqueous NaOH solution were employed.

### Preparation of drug-containing raw granules

After 3300 g of core granules ("Nonpareil 101, 20-24#" produced by Freund Corporation) were placed in a centrifugal fluidizing coating apparatus ("CF coater CF-360" produced by Freund Corporation), 1200 g of theophylline, 2400 g of salazosulfapyridine and 36 g of hydroxypropyl cellulose ("HPC HPC-SSL" produced by Nippon Soda) were fed while a 5 wt% solution (ethanol/water (weight ratio of 5/5))of hydroxypropylmethyl cellulose ("HPMC TC-5E" produced by Shin-Etsu Chemical Co., Ltd.) was sprayed. As a result, granules were prepared. Each of these raw granules was found to have a theophylline content of 0.1 mg and a salazosulfapyridine content of 0.2 mg.

### Example 1

Enteric coating was applied to the drug-containing raw granules prepared in the above-described manner. The enteric coating solution was obtained by mixing 30 parts by weight of talc with 100 parts by weight of hydroxypropylmethyl cellulose acetate succinate (HPMCAS, produced by Shin-Etsu Chemical and containing 23.9 wt% of a methoxyl group, 7.6 wt% of a hydroxypropoxyl group, 11.4 wt% of an acetyl group and 6.2 wt% of a succinoyl group) and adding a mixed solution of ethanol/water (weight ratio of 8/2) thereto in an amount sufficient to adjust the solid concentration of the hydroxypropylmethyl cellulose acetate succinate to be 7% by weight.

The drug-containing granules (360 g) obtained in the above-described manner were placed in a rotary fluidized-bed coater ("Multiplex MP-01" produced by Powrex) and the coating composition was applied to the raw granules at a supply air temperature of 60°C, a discharge air temperature of 42°C, revolutions of 200 rpm, a spray rate of 15 g/min and a spray air rate of 30 L/min so as to adjust the solid content in the coating composition to be 39 wt% based on the weight of the raw granules.

### Example 2

In a similar manner to Example 1 except that the hydroxypropylmethyl cellulose acetate succinate was replaced by hydroxypropylmethyl cellulose acetate succinate having different substituent contents (HPMCAS containing 24.0 wt% of a methoxyl group, 7.5 wt% of a hydroxypropoxyl group, 13.0 wt% of an acetyl group and 5.2 wt% of a succinoyl group), coating was performed.

### Example 3

In a similar manner to Example 1 except that the hydroxypropylmethyl cellulose acetate succinate was replaced by hydroxypropylmethyl cellulose acetate succinate having different substituent contents (HPMCAS containing 24.2 wt% of a methoxyl group, 7.5 wt% of a hydroxypropoxyl group, 12.5 wt% of an acetyl group and 4.3 wt% of a succinoyl group), coating was performed.

### Example 4

In a similar manner to Example 1 except that the hydroxypropylmethyl cellulose acetate succinate was replaced by hydroxypropylmethyl cellulose acetate succinate having different substituent content (HPMCAS containing 24.3 wt% of a methoxyl group, 7.7 wt% of a hydroxypropoxyl group, 12.5 wt% of an acetyl group and 4.1 wt% of a succinoyl group), coating was performed.

### Example 5

In a similar manner to Example 1 except that the hydroxypropylmethyl cellulose acetate succinate was replaced by a copolymer of methyl methacrylate and methacrylic acid (methacrylic acid copolymer S "Eudragit S", having a methacrylic acid content of 29.2 wt% and produced by Rohm and Haas), coating was performed.

### Example 6

In a similar manner to Example 1 except that the solid content weight in the coating composition was adjusted to 26 wt% based on the weight of the raw granules, coating was performed.

### Example 7

In a similar manner to Example 3 except that the amount of the talc was changed to 3 parts by weight and the solid content in the coating composition was adjusted to 30.9 wt% based on the weight of the raw granules, coating was performed.

### Example 8

An aqueous solution of a composition containing 100 parts by weight of HPMCAS similar to that used in Example 1, 30 parts by weight of talc, 35 parts by weight of triethyl citrate and 0.3 part by weight of sodium lauryl sulfate was prepared while adjusting the solid concentration of the HPMCAS to be 7 wt%. The drug-containing granules (360 g) similar to those employed in Example 1 were placed in a rotary fluidized-bed coating apparatus and the coating composition was applied to the raw granules at a supply air temperature of 80°C, a discharge temperature of 44°C, revolutions of 200 rpm, a spray rate of 7.5 g/min and a spray air rate of 30 L/min so as to adjust the solid content in the coating composition to be 49.6 wt% based on the weight of the raw granules.

### Comparative Example 1

In a similar manner to Example 1 except that the hydroxypropylmethyl cellulose acetate succinate was replaced by hydroxypropylmethyl cellulose acetate succinate (HPMCAS produced by Shin-Etsu Chemical Co., Ltd. and containing 23.0 wt% of a methoxyl group, 7.2 wt% of a hydroxypropoxyl group, 9.5 wt% of an acetyl group and 11.1 wt% of a succinoyl group), coating was performed.

### Comparative Example 2

In a similar manner to Example 1 except that the hydroxypropylmethyl cellulose acetate succinate was replaced by hydroxypropylmethyl cellulose acetate succinate (HPMCAS produced by Shin-Etsu Chemical Co., Ltd. and containing 22.3 wt% of a methoxyl group content of, 7.1 wt% of a hydroxypropoxyl group, 7.8 wt% of an acetyl group and 15.4 wt% of a succinoyl group), coating was performed.

### Comparative Example 3

In a similar manner to Example 1 except that the hydroxypropylmethyl cellulose acetate succinate was replaced by a methyl methacrylate / methacrylic acid copolymer (methacrylic acid copolymer L "Eudragit L" having a methacrylic acid content of 48.1 wt% and produced by Rohm and Haas), coating was performed.

### Evaluation 1: In vitro dissolution test

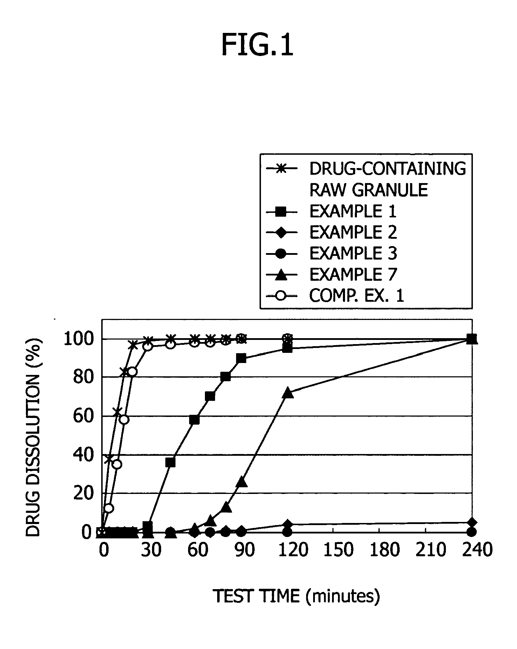

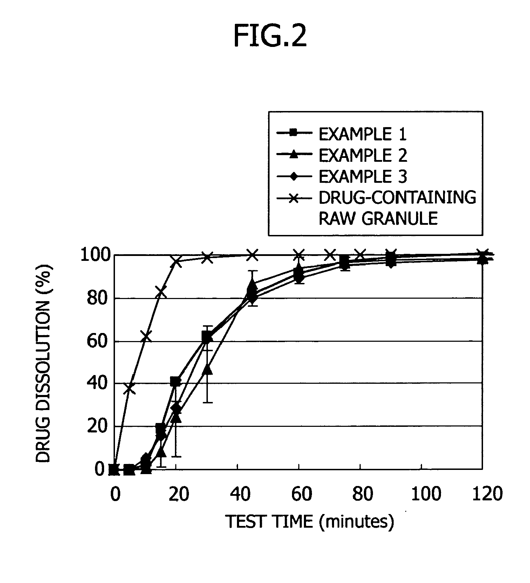

Dissolution properties of the drug from the coating preparations prepared in Examples 1 to 8 and Comparative Examples 1 to 3 were evaluated in accordance with the Japanese Pharmacopoeia Dissolution Test (paddle method, 50 rpm). The Japanese Pharmacopoeia first fluid (pH 1.2), Japanese Pharmacopoeia second fluid (pH 6.8) and a phosphate buffer of pH 7.6 were employed for the test. The results are shown in Table 2. The dissolution profiles of the drug-containing raw granules and the enteric-coated preparations obtained in Examples 1 to 3 and Comparative Example 1 in the Japanese Pharmacopoeia second fluid are shown in FIG. 1 as typical examples. In addition, the dissolution profiles of the drug-containing raw granules and the enteric-coated preparations obtained in Examples 1 to 3 in a phosphate buffer of pH 7.6 are shown in FIG. 2.

As is evident from the results shown in Table 2, the preparations obtained in Example 1 to 8 did not disintegrate even after 2 hours in the Japanese Pharmacopoeia first fluid which meets a simulated stomach acid condition. Accordingly, dissolution of the drug was not observed. In addition, they satisfied the target lag time in the Japanese Pharmacopoeia second fluid which meets a simulated small-intestinal environment.

FIG. 2 has revealed that at pH 7.6, the dissolution of the drug was observed after the lag time of from 5 to 10 minutes regarding any one of the preparations obtained in Examples 1 to 3. The pH value at the lower small intestine is usually near 7.6. The enteric-coated preparation obtained in Example 2 or 3, which did not release the drug at pH 6.8 which is an environment close to that in the upper and middle small intestines in FIG. 1, showed the dissolution at pH 7.6. Thus, it can be presumed that these preparations can definitely have site-specific dissolution of the drug after being delivered to the vicinity of the lower small intestine.

### Evaluation 2: In vivo test

After Wistar male rats (each, about 250 g) were fasted for twenty-four hours, 0.5 mL of water and one enteric-coated preparation obtained in each of Examples 1 to 4, 7 and 8 and Comparative Examples 1 and 2 were administered orally.
After administration, the blood was collected from the jugular vein every hour for 8 hours. The theophylline and sulfapyridine concentrations in the plasma thus collected were measured by HPLC. A similar investigation was performed using an aqueous solution of the drug. The test was performed while setting the number of test specimens at n=5. Their average was provided as an analytical value.

After arrival at the large intestine, the azo group of salazosulfapyridine, one of the active ingredients, is reduced by microorganism and decomposed into sulfapyridine and 5-aminosalicylic acid. The points of time when the theophylline and sulfapyridine were detected for the first time in the blood were designated as a starting time of preparation disintegration and drug dissolution, and an arriving time at the large intestine, respectively.

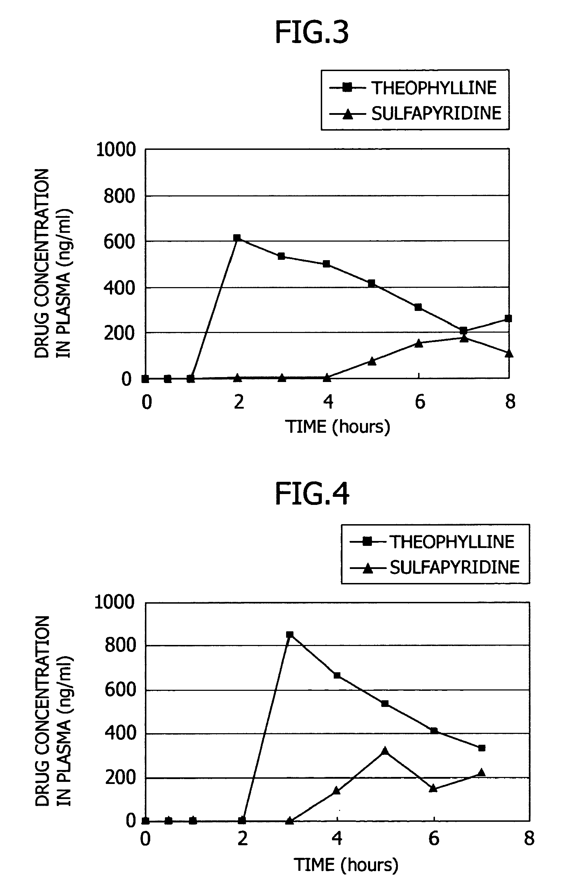

The results are shown in Table 3. As typical examples, the blood level profiles of typical test specimens obtained in Example 1 and Example 3 are shown in FIGS. 3 and 4, respectively. As is evident from FIG. 3, it has been understood that even if the drug is not released in the Japanese Pharmacopoeia second fluid even after 2 hours in the in vitro dissolution test, in vivo release of the drug can be accomplished when the material satisfies the dissolution properties under specific conditions. The following can be presumed as shown in these results and based on consideration of the time of starting to detect sulfapyridine being 4 or 4.5 hours and difference from the time of starting to detect the theophyllin in the blood level, wherein the time of starting to detect sulfapyridine means the arrival of the preparation at the large intestine. With respect to the pharmacokinetic (Table 3) of the enteric-coated preparations obtained in Examples 1 to 4, 7 and 8 and Comparative Examples 1 and 2, the drug was delivered to the middle small intestine with the difference of about 2 hours in Examples 1, 2 and 8, to the lower small intestine with the difference of within 1 hour in Examples 3, 4 and 7, and to the stomach and the upper small intestine with the difference of 3 hours or more in Comparative Examples 1 and 2.

**Table 1**

| | substituent (wt%) | | | | | acetyl/succinoyl | solubility of film | | | | | |
|---|---|---|---|---|---|---|---|---|---|---|---|---|
| | material | methoxyl | hydroxypropoxyl | acetyl | succinoyl | | pH | | | | | 0.1N NaOH |
| | | | | | | | 1.2 *1 | 6.8 *1 | 7.6 *2 | 8.0 *2 | 8.6 *3 | |
| Example 1 | HPMCAS | 23.9 | 7.6 | 11.4 | 6.2 | 1.84 | >120 | >120 | 30 | 20 | 15 | 3 |
| Example 2 | HPMCAS | 24.0 | 7.5 | 13.0 | 5.2 | 2.50 | >120 | >120 | 76 | 75 | 23 | 4 |
| Example 3 | HPMCAS | 24.2 | 7.5 | 12.5 | 4.3 | 2.91 | >120 | >120 | >120 | >120 | 52 | 8 |
| Example 4 | HPMCAS | 24.3 | 7.7 | 12.5 | 4.1 | 3.05 | >120 | >120 | >120 | >120 | >120 | 10 |
| Example 5 | Eudragit S | | | | | | >120 | >120 | 15 | 13 | 8 | 3 |
| Example 6 | HPMCAS | 23.9 | 7.6 | 11.4 | 6.2 | 1.84 | >120 | >120 | 30 | 20 | 15 | 3 |
| Example 7 | HPMCAS | 24.2 | 7.5 | 12.5 | 4.3 | 2.91 | >120 | >120 | >120 | >120 | 52 | 3 |
| Example 8 | HPMCAS | 23.9 | 7.6 | 11.4 | 6.2 | 1.84 | >120 | >120 | 30 | 20 | 15 | 3 |
| Comp.Ex.1 | HPMCAS | 23.0 | 7.2 | 9.5 | 11.1 | 0.86 | >120 | 8 | 6 | 6 | 5 | 3 |
| Comp.Ex.2 | HPMCAS | 22.3 | 7.1 | 7.8 | 15.4 | 0.51 | >120 | 30 | 20 | 10 | 8 | 3 |
| Comp.Ex.3 | Eudragit S | | | | | | >120 | >120 | 10 | 15 | 10 | 5 |

| | | | | | | | | | | | | |
|---|---|---|---|---|---|---|---|---|---|---|---|---|
| *1 A test fluid in Dissolution Test of the Japanese Pharrmacopoeia *2 phosphate buffer in US Pharmacopoeia *3 borate buffer in US Pharmacopoeia | | | | | | | | | | | | |

**Table 2**

| | Dissolution Test | |
|---|---|---|
| | Japanese Parmacopoeia first fluid 2 hours later | Japanese Pharmacopoeia second fluid lag time *4 (minutes) |
| Example 1 | no dissolution | 30 |
| Example 2 | no dissolution | 80 |
| Example 3 | no dissolution | 240 |
| Example 4 | no dissolution | 240 |
| Example 5 | no dissolution | 60 |
| Example 6 | no dissolution | 60 |
| Example 7 | no dissolution | 60 |
| Example 8 | no dissolution | 30 |
| Comp.Ex.1 | no dissolution | 5 |
| Comp.Ex.2 | no dissolution | 5 |
| Comp.Ex.3 | no dissolution | 5 |

| | | |
|---|---|---|
| *4 An inverval between samplings is 5 to 30 minutes. The lag time is defined as a time requried for starting to detect the first drug dissolution. | | |

**Table 3**

| | time required for the prepartion to start disintegration and for the drug to start dissolution : time required to detect theophylline T(the) (hours) | time required for the preparation to reach the large intestine : time required to detect sulfapyridine T(sul) (hours) | difference of times required to detect sulfapyridine and theophylline : T(sul) - T(the) (hours) |
|---|---|---|---|
| Example 1 | 2.0 | 4.5 | 2.5 |
| Example 2 | 2.0 | 3.5 | 1.5 |
| Example 3 | 2.8 | 3.5 | 0.7 |
| Example 4 | 3.0 | 3.5 | 0.5 |
| Example 7 | 2.3 | 3.3 | 1.0 |
| Example 8 | 2.0 | 4.5 | 2.5 |
| Comp.Ex.1 | 0.5 | 4.5 | 4.0 |
| Comp.Ex.2 | 0.5 | 4.0 | 3.5 |

## Claims

1. An enteric-coated preparation for delivering a drug to a site within the small intestine in a site-specific manner, comprising a drug-containing preparation and an enteric coating material which covers the preparation,
wherein, in Dissolution Test as specified in the Japanese Pharmacopoeia Fourteenth Edition, no dissolution of the drug is observed in the Japanese Pharmacopoeia first fluid (pH 1.2) while dissolution of the drug is observed in the Japanese Pharmacopoeia second liquid (pH 6.8) 20 minutes or later from start of the Dissolution Test.

2. The enteric-coated preparation according to Claim 1, wherein the enteric coating material is selected from the group consisting of hydroxypropylmethyl cellulose acetate succinate, hydroxypropylmethyl cellulose phthalate, methacrylic acid copolymer and carboxymethylethyl cellulose.

3. The enteric-coated preparation according to Claim 2, wherein the enteric coating materiel is hydroxypropylmethyl cellulose acetate succinate having a succinoyl group content of 0.1 to 10% by weight, or methacrylic acid copolymer having a methacrylic acid content of 25 to 34.5% by weight.

4. The enteric-coated preparation according to Claim 2, wherein the enteric coating material is hydroxypropylmethyl cellulose acetate succinate having a succinoyl group content of 0.1 to 10% by weight, an acetyl group content of 5 to 14% by weight, and keeping a ratio of the acetyl group content to the succinoyl group content from 1.25 to 5.
